# EUROPEAN PATENT APPLICATION

(11) **EP 4 843 560 A1**
(43) Date of publication of application: **25.11.2026**
(21) Application number: 26179600.7
(22) Date of filing: 19.05.2026
(51) Int. Cl.: A61K 8/92, A61K 8/34, A61K 8/96, A61K 8/97, A61Q 19/10

(54) **BLACK LIQUOR AS AN INGREDIENT FOR COSMETIC COMPOSITIONS**

(30) Priority: 20.05.2025 IT 202500011308
(71) Applicant: Fluos S.A.S. Di Giuseppe Chiaradia & C., 21052 Busto Arsizio (VA) (IT); Università degli Studi di Torino, 10124 Torino (IT)
(72) Inventor: CHIARADIA, Giuseppe, Busto Arsizio (VA) (IT); CAVALLI, Roberta, Alessandria (IT); SCOMPARIN, Anna, Torino (IT); MOLINAR, Chiara, Balangero (TO) (IT); COLOMBO, Dario, Busto Arsizio (VA) (IT); BASTIAENSEN, Erik, Kapellen (BE); TURESSO, Caterina, Busto Arsizio (VA) (IT)
(74) Representative: Giugni, Diego

(57) **Abstract**

The present invention refers to a new natural ingredient for cosmetic compositions. In particular, the invention refers to an ingredient of plant origin having different properties, both cosmetic and functional for the cosmetic composition itself. The ingredient is the so-called "Black liquor" of totally vegetable origin that can be used as it is in very low quantities with surprising performance compared to conventional additives of a synthetic nature or derived from petroleum.

## Description

### FIELD OF THE INVENTION

The present invention refers to a new natural ingredient for cosmetic compositions. In particular, the invention refers to an ingredient of plant origin having different properties, both cosmetic and functional for the cosmetic composition itself. The ingredient is the so-called "Black Liquor" of totally vegetable origin, specified below, which can be used as it is in very low quantities with similar or in some cases better performance than conventional synthetic or petroleum-derived ingredients.

### BACKGROUND OF THE INVENTION

Cosmetic compositions for hygiene and skin care are usually water-based emulsions that contain chemical ingredients that provide specific functionality or properties to the composition, such as surfactants, emollients, emulsifiers, dispersants, antioxidants, preservatives, UV filters, and fragrances. The skin of human beings is a layer of tissue that covers the body and performs the functions of protecting against the penetration of external pathogens or UV radiation and regulating the diffusion of water to the outside and body temperature.

The harmful effects of ionic and even non-ionic surfactants on the integrity of the stratum corneum have long been widely known, due to the solubilizing action of the lipids of the "skin barrier", but in recent years increasingly in-depth scientific research is revealing that some ingredients used in cosmetic products have harmful effects on human health and ecosystems. The toxicological characteristics of some of the questioned ingredients commonly used as ingredients in cosmetic formulations are reported below.

PFAS (perfluoroalkyl substances): exposure to these substances can have several negative health effects, including an increased risk of cancers (such as kidney, testicular and breast cancer), liver enzyme alterations, fertility problems, obesity, endocrine disorders and immune system dysfunction. In addition, they may be associated with developmental problems in children and an increased risk of hypertension and pre-eclampsia in pregnancy.

Petrolatum: these are well-known substances, of petroleum derivation and are occlusive (they do not allow the skin to breathe) and are not dermo-compatible; on the label they are reported with the words "Paraffinum liquidum, Petrolatum, Mineral Oil, Vaseline, Ceresin, Ozokerite".

Silicones: these substances are also not dermocompatible and create a film on the skin and hair with a fake "moisturizing" effect; on the label they are reported with the words: "Dimethicone, Dimethiconol, Polydimethicone, Cyclopentasiloxane, Cyclohexasiloxane, Quaternium-80, Amodimethicone, Cyclomethicone, Trimethylsiloxysilicate".

Preservatives: there are allergenic preservatives such as Methyl chloro isothiazolinone and Methyli sothiazolinone; we also find them in the form of parabens whose family of preservatives is known for its bad reputation as they create intolerances and are potential endocrine disruptors, i.e. able to interfere with our hormonal system; on the label they are reported with the words "Isobutyl Paraben, Butyl Paraben, Methyl Paraben, Propylparaben, Benzyl Paraben, Ethyl Paraben. Among the preservatives, formaldehyde releasers are responsible for releasing formaldehyde (a substance considered carcinogenic with certainty); on the label they are reported with the wording: Sodium Hydroxymethyl Glycinate, Bronopol, Diazolidynyl Urea, Benzylhemiformal, Imidiazolidynyl Urea, Quaternium-15, DMDM Hydantoin.

Antimicrobials: synthetic antioxidants with a high risk of skin sensitization such as Butylated hydroxyanisole (BHA) and Butylated hydroxytoluol (BHT), or toxic antibacterial substances such as Triclosan, which can also cause antibiotic resistance.

Ethoxylated surfactants: synthetic substances that facilitate the fluidity of the product; PEGs and PPGs can be contaminated with amounts of ethylene oxide and 1,4-dioxane, both carcinogens; on the label they are reported with the words: "Polyethyleneglycole (PEG), Dipolyhydroxystearate, PEG6, PEG20, PEG 75, PEG-40 Hydrogenated Castor Oil, Propylen Glycol, Butylen Glycol, Polypropylene Glycol, Ethylene Glycol".

Ionic surfactants: the interaction of surfactants, in particular anionic ones, with the lipids and proteins that make up the stratum corneum of the skin, leads to several drawbacks including: skin dryness, erythema, irritation, itching, stiffness. This is due to the fact that surfactants tend to solubilize not only grease and oily substances in general, but also lipids ordered according to a lamellar structure that constitutes the skin barrier within the stratum corneum. In the skin barrier, lipid molecules are arranged compactly: micro-photographs indicate that they are placed side by side neatly and perpendicular to the surface of the skin. If the lamellae structure is damaged or destabilized, the following disadvantages occur: 1) the penetration of foreign molecules, such as the laureth sulfate anion or stearate, is favored beyond the stratum corneum towards the inner layers of the epidermis; 2) the process of desquamation of the surface cells is facilitated. As a result, more intense activity is required by internal cells to synthesize new bio-molecules in order to restore the eroded stratum corneum; on the label they are reported with the words "Sodium Lauryl Sulfate (SLS), Amonium Lauryl Sulfate, TEA-Lauryl Sulfate, Magnesium Laureth Sulfate, MEA Laureth Sulfate, Sodium Laureth Sulfate (SLES) and Amonium Laureth Sulfate".

Oleamides: substances that penetrate the skin to form nitrosamines (carcinogens); on the label they are reported with the words: Cocamide DEA, Oleamide DEA, Lauramide DEA, Cocamide TEA, Oleamide TEA, Lauramide TEA, Cocamide MEA, Oleamide MEA, Lauramide MEA, Linoleamide DEA, Myristamide DEA, Stearamide DEA, DEA-Cethyl Phosphate, DEA-Oleth-3 Phosphate, TEA-Lauryl Sulfate Thiethanoamine.

In the publication "The 500 Dalton rule for the skin penetration" (Jan D. Bos and Marcus Meinardi, 2000, Exp Dermatol 2000: 9: 165-169), the authors state that compounds with a molecular weight of less than about 500 Daltons are easily absorbed by the stratum corneum of the skin and penetrate to the inner layers, consisting of the epidermis and dermis. Larger molecules can cross the stratum corneum only if they have certain characteristics or associated with other lipid-derived substances called liposomes.

The arguments in favor of this "500 Dalton rule" are due to the fact that all common contact allergens are less than 500 Daltons, larger molecules are not known as contact sensitizers, and that all known topical drugs used in transdermal drug delivery systems are below 500 Daltons.

Therefore, due to the increasing attention to personal care not only from the point of view of hygiene and aesthetics but also and above all from the point of view of health, there is an increased attention from consumers towards hygiene and skin care products that are free of harmful chemicals that can penetrate through the skin damaging it. In addition, government authorities tend to direct the research activities of pharmaceutical companies towards the valorization of sustainable raw materials, and which minimize the impact of cosmetic products on biodiversity.

Among the various products of natural origin that have been tested as substitute ingredients for the substances mentioned above, lignin has been studied in order to exploit above all the anti-oxidant properties of the aromatic components contained in it. The structural characteristics of lignin are examined below.

### LIGNIN

Wood is mainly composed of cellulose, hemicellulose and lignin. The remaining components, less than 5% by weight, include what are called "extractives"; they consist of resins, fatty acids, and terpenes. Hemicellulose is a complex polysaccharide, consisting of pentose sugars (such as arabinose and xylose) and hexose sugars (such as mannose and glucose). Cellulose which is mainly made up of glucose.

Lignin belongs to the class of polyphenols, which are structures consisting of one or more aromatic rings containing one or more hydroxyl groups. Depending on the number of phenolic rings and the elements that make up the structures that connect them, it is possible to distinguish different classes, such as phenolic acids, flavonoids, stilbenes and lignans. These substances are recognized for their antioxidant, anti-inflammatory, antibacterial, antifungal, antiviral, antiallergenic, anticancer and anticoagulant effects. In addition, plant polyphenols are considered significant for maintaining healthy skin due to their effects on hydration, smoothness, softness, soothing and astringency.

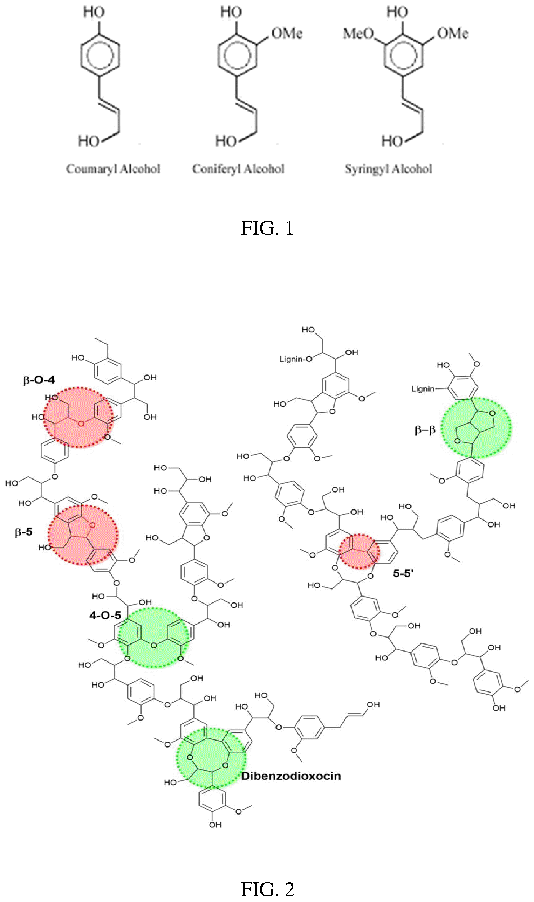

Lignin is mainly found in the cell wall of certain plant cells. The lignin molecule has a high molecular weight and is obtained from the union of different phenyl-propyl alcohols. They are (see Fig. 1):
- p-coumaryl alcohol (4-hydroxycinnamyl alcohol);
- coniferyl alcohol (4-hydroxy-3-methoxycinnamyl alcohol);
- synapyl alcohol (4-hydroxy-3,5-dimethoxycinnamylic alcohol).

The bonds that connect lignin alcohols are the β-O-4 ether groups (the most important, 40-60% of the total and easiest to break) and the α-O-4 group, which is less frequent and in any case labile in an alkaline environment. Carbon-carbon bonds include β-β (resinol), β-5 (phenyl-coumaran) and 5-5 (biphenyl). Fig. 2 shows some of the internal bonds of lignin.

Lignin, hemicellulose and cellulose form unique and complex structures in wood. Table 1 below shows the typical compositions of hardwood (broad-leaved trees such as oak or beech), softwood (conifers such as pine or fir) and woody biomass such as wheat straw.

**TABLE 1**

| Typical compositions of woody biomass | | | | |
|---|---|---|---|---|
| | Lignin % wt | Cellulose % wt | Hemicellulose % wt | Extractives % wt |
| Hardwood | 18-25 | 40-50 | 25-35 | 3 |
| Softwood | 25-35 | 40-45 | 20-30 | 3 |
| Wheast straw | 15-20 | 33-40 | 20-28 | 3 |

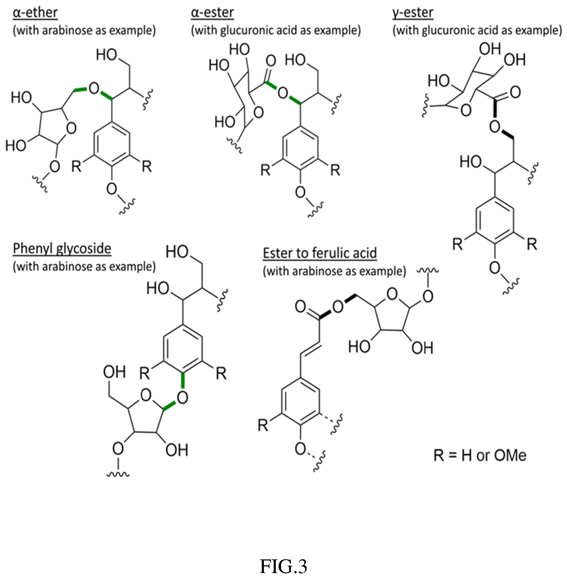

The most common lignin-carbohydrate bonds (LCCs) are: α-ether, α-ester, γ phenyl-glucoside, ferulic acid ester (see Fig. 3).

Numerous studies report the presence of LCC in native biomass materials in coniferous, deciduous and non-woody plants. Due to its strong binding, the presence of LCC affects the overall extraction of lignin and carbohydrates. For example, a low yield of the Kraft pulping process is related to the challenge of breaking lignin-carbohydrate bonds in hardwood species, which can be attributed to the alkaline stability of LCC bonds. In addition, the formation of lignin-hemicellulose bonds in kraft pulp has been suggested. Several studies confirm the existence of lignin-hemicellulose bonds in softwood and Kraft wood pulp.

Lignin has numerous properties and activities listed below.

### ANTIOXIDANT ACTIVITY

Several studies have shown that lignin possesses antioxidant properties and these properties are believed to be conferred by the hydroxyl and methoxyphenolic groups of the aromatic rings. The antioxidant property makes lignin a good alternative to replace synthetic cytotoxic antioxidants such as butylated hydroxytoluene (BHT) or butylated hydroxyanisole (BHA), which are widely used in industries such as pharmaceuticals, cosmetics, food and plastics. The impetus for alternatives to synthetic antioxidants lies in the fact that several studies have shown their harmful effect at the molecular and cellular level, even at low concentrations, as explained above.

Depending on the biomass source and cellulose production process, lignins vary in their structure and possess different antioxidant and UV-absorbing properties.

The properties of lignin can be used to prevent oxidation reactions in biofuels, animal feed and polymer composite materials. In addition, the ability of phenolic groups to eliminate free radicals gives lignin excellent properties such as increasing the thermal and oxidative stability of polymers in which lignin is added as a component.

### ANTIMICROBIAL ACTIVITY

Among the commercially available technical lignins, lignosulfonates and Kraft lignins have been reported to exhibit antibacterial, antifungal, antiviral and prebiotic activities. In addition, recent research on the development of novel biopolymers with microbicidal properties has led to the development and evaluation of lignin-derived biomaterials, including dealkaline lignin-based hydrogels, nanocomposite fibers made from alkaline kraft lignin and nanoparticles synthesized by dissolving alkaline kraft lignin into ethylene glycol.

The publication "The Antimicrobial Properties of Technical Lignins and Their Derivatives-A Review (2024, Polymers, Diana Carolina Reyes, Zhengxin Ma and Juan Jose Romero)" summarizes the studies carried out so far on lignin extracted from different types of biomass and with different processes.

Nowadays, the industry that produces the largest amount of lignin is the production of cellulose pulp by processes of extraction of lignin from wood and other biomasses.

### USES OF PRECIPITATED LIGNIN IN COSMETICS

Although numerous scientific studies carried out in vitro have recognized the antioxidant and antimicrobial properties of lignin, the use of this polyphenol in compositions for hygiene and skin care is modest.

Niche applications are known in formulations for "sun care" products to exploit UV protections and in make-up as a co-emulsifier thanks to the stabilizing action of lignin nano-particles (LNP).

A number of methods have been adopted to synthetise LNPs, including colloid production by self-assembly, dissolution of lignin in an organic solvent and dialysis (solvent exchange), acid precipitation, carbon dioxide saturation, high shear and pressure homogenisation and ultrasonic technique.

The color of the lignin, the performance of the sunscreen and other functional properties are then strongly influenced by the size and morphology of the particles. Colloidal lignin particles have additional advantages for cosmetic applications, such as increasing dispersibility in water and improving functional properties.

However, the application of lignin nano-particles in skin care and hygiene applications is hampered by the fact that lignin generally appears in the form of solid nano-particles and not as a dissolved or emulsified liquid component.

Depending on the application, the lignin placed on the market can be:
- functional dust (masks, detergents);
- colloidal dispersion;
- liquid resolubilized or derivatized in a solvent (not in water);
- encapsulated (for improved color and stability).

To obtain lignin for cosmetic use, it is first necessary to make an industrial production of lignin in solid powdery form and then to do further treatments to obtain nanoparticles, dispersions or solubilized lignin in solvent.

The subsequent stages of production are: a) precipitation of lignin by acidification of the Black Liquor; b) filtration; c) Washing; d) drying; e) purification. After all these treatments, lignin loses most of the carbohydrate component and the result is a predominantly lignin, polyphenolic and aromatic material, chemically distinct from the LCCs present in Black Liquor.

During the precipitation and washing phases, an initial compositional selection takes place:
- the ester-type bonds in LCC are broken by hydrolysis and remain largely the most stable (such as benzyl ethers and benzyl glucoside);
- free and poorly associated carbohydrates remain in solution;
- the washing removes soluble carbohydrates, salts and small molecules.
So already after the first precipitation phase, the precipitated lignin is no longer the "lignin of the Black Liquor", but a selected fraction.

After drying/purification, the typical composition becomes:
- 90-95%WT poly-aromatic lignin;
- residual carbohydrates, traces or at most a few %.
Therefore, the LCC complexes are degraded almost entirely after precipitation by acidification and drying. Carbohydrates remain as residues in percentages of up to a few percent.

In the case of the production of lignin-based sunscreen products, the following steps are required:
- selective dissolution;
- self-assembly/nano-precipitation;
- further exclusion of hydrophilic species (carbohydrates).

Thus lignin nanoparticles are dominated by the lignin aromatic matrix with phenolic groups precipitating during acidification. A significant portion of the low molecular weight polyphenols and highly hydrophilic LCC complexes not precipitated after acidification treatment remain solubilized in the aqueous phase and are lost.

The document CN85107583A describes the use in cosmetics of lignin degradation products extracted from Black Liquor. In particular, those products are the result of precipitation from Black Liquor, i.e. separation from it.

The document CN101589134B describes a process for separating Black Liquor from wood pellets by means of alkyl alcohol alkoxylates.

The WO2023/037013 document describes a method for obtaining an antioxidant composition from lignin derivatives or Black Liquor.

The paper "Formulation of sunscreen cream that contain lignin and their manufacturing process", Thomson Scientific, London, UK; AN 2025-23582S, XP002819418, describes a formulation for a lignin-containing sunscreen extracted from Black Liquor.

### SUMMARY OF THE INVENTION

The technical problem underlying the present invention is therefore to find a lignin-based ingredient that overcomes the drawbacks of the isolated lignin currently used in the form of nano-particles in the preparation of cosmetic products.

This problem is solved by the use of the so-called Black Liquor, obtained as a by-product of woodworking.

A first object of the present invention is therefore the use of Black Liquor as an ingredient in cosmetic compositions.

A second object is a cosmetic composition comprising Black Liquor as a stabilizing, emulsifying, dispersant, antioxidant, antimicrobial, preservative ingredient.

A third object is a cosmetic composition comprising Black Liquor as a cleansing, make-up remover, cleanser, anti-ageing, antimicrobial, antioxidant agent.

An additional object is the use of Black Liquor in a cosmetic composition for cleansing skin and hair.

### BRIEF DESCRIPTION OF THE FIGURES

Further characteristics and advantages of the use of Black Liquor in cosmetics according to the present invention will become more evident from the following description of some embodiments given by way of example only, with reference to the attached figures, in which:
- Figure 1 represents the structural formula of the three phenyl propyl alcohols of which lignin is composed;
- Figure 2 represents the structural formula of some of the internal bonds of lignin;
- Figure 3 represents the structural formula of the most common types of lignin-carbohydrate bonds in woody biomass.

### DETAILED DESCRIPTION OF THE INVENTION

It has been surprisingly discovered that the aqueous mixture commonly referred to as Black Liquor obtained from the processes of extracting lignin from wood can be used in cosmetic compositions as an ingredient with antioxidant, antimicrobial, preservative, emulsifier and dispersant properties.

Black Liquor is defined as the aqueous by-product that is obtained during wood processing to free cellulose fibers from lignin, hemicellulose and other wood components in order to obtain the so-called cellulose pulp. The lignin in Black Liquor appears both in free form as a polyphenol and as lignin-carbohydrate complexes (LCCs).

Studies on the absorption of lignin and its complexes on the skin with carbohydrates contained in Black Liquor according to the present invention have shown that the addition of Black Liquor in a cosmetic formulation allows, in topical applications, to convey lignin and lignin carbohydrate complexes (LCC) in dissolved or emulsified form, making it easier for them to penetrate in the inner layers of the skin, together with polyphenols with a lower molecular weight, compared to lignin in the state of solid particles with a high molecular weight, and making it easier to interact with the other components of the cosmetic composition.

The properties that have emerged from the studies on the applications of Black Liquor in cosmetics, reported as examples in this description, make it possible to eliminate or quantitatively reduce toxic or harmful ingredients of petroleum origin, which are not environmentally friendly and have properties similar to those of lignin, such as anti-aging or anti-oxidant properties.

The effectiveness of the properties conferred by Black Liquor to cosmetic compositions is related to the amount added in the formulation and the type of property required. Experimental data indicate that a concentration of at least 0.1% wt (by weight) of lignin in the formulation of the cosmetic product is sufficient to confer on it the properties claimed in the present invention.

The lignin content in the Black Liquor must be sufficient to allow the lignin of the components of the cosmetic product to have at least a concentration of 0.1% wt on the total weight of the cosmetic composition.

Considering that in formulations in cosmetic products the water content generally reaches up to 90% wt by weight, the minimum concentration of lignin in Black Liquor must also be a minimum of 0.1% wt so that a cosmetic composition containing a high concentration of Black Liquor can act effectively.

During the preparation of cosmetic compositions, Black Liquor can be added to compositions according to typical operating methods without special conditions being required.

Black Liquor is an aqueous solution ranging in color from dark brown to black containing, together with the chemicals used for wood pulping, substances extracted from wood, such as lignin, polysaccharides and their degradation products and small quantities of an oily resin called turpentine, containing terpenes, higher alcohols and resin acids. In the following, the chemical characteristics of Black Liquor obtained from the processes of extraction of lignin from woody biomass will be briefly described.

### BLACK LIQUOR FROM THE EXTRACTION OF LIGNIN FROM BIOMASS

The extraction of lignin from biomass can be carried out in many different ways. The most common methods are the Krat and sulfite processes, both of which produce sulfur-containing lignin. Sulphur is undesirable, for example, if lignin is intended to be used as a fuel, since in such cases both lignin and flue gases from the combustion of the fuel must undergo costly desulphurisation. There are processes that produce sulfur-free lignin, such as the soda pulping process and various processes of Organosolv, which use various solvents such as acetone, methanol, ethanol, butanol, ethylene glycol, formic acid, and/or acetic acid. Other methods, such as acid hydrolysis, use concentrated acids to hydrolyze carbohydrates, leaving lignin as an insoluble residue.

In alkaline processes, cellulose fibers are dissociated from lignin by chemical reactions. These reactions take place in a pressurized digester, where wood chips or fibers are heated and cooked with cooking liquor, which is basically composed of NaOH (sodium hydroxide). Specifically in the Kraft process, sodium sulfide (Na₂S) is added to the digester to improve the dissociation of lignin from cellulose fibers, accelerating the wood cooking operation and increasing the mechanical strength of the pulp. The products resulting from digester reactions are cellulose pulp and Black Liquor.

### KRAFT PROCESS

Nowadays, the industry that produces the largest amount of lignin is the pulp and paper industry. In the Kraft wood pulping process, cellulose fibers are separated from hemicellulose and lignin by cooking the wood in an alkaline solution of NaOH with the addition of Na₂S. The resulting solution is commonly called Black Liquor in the paper industry, and consists of a complex mixture of lignin (and related substances), degradation products of hemicelluloses, soaps (originating from lipophilic extracts) and inorganic salts. In the production of one ton of pulp, about 7 tons of Black Liquor are produced, which is largely burned to recover pulping chemicals and to produce energy. However, a part of Black Liquor can be used to extract lignin in the form of nano-particles. However, only less than 2% of the lignin produced by the pulp industries is used for industrial applications, resulting in a serious waste of valuable aromatic resources.

Currently, the kraft process is the world's predominant method for cellulose production, with up to 90% of cellulose used in all paper mills, due to its advantages over other pulping methods. These benefits mainly include obtaining stronger fibres, the ease with which it can be applied to all wood species and the recovery of spent chemicals. Basically, Kraft cellulose production consists of four stages: wood preparation, pulping, bleaching, and chemical recovery. More specifically, the logs are first debarked and turned into shavings, which are then digested with an aqueous solution (called White Liquor) with 15-20% sodium sulfide (Na₂S) and sodium hydroxide (NaOH) at high temperatures (145-170 °C) and pressures (6-7 bar) for 2-3 hours. During this step, the cellulose fibers (pulp) are separated from the solubilized compounds, which make up a liquid fraction called Weak Black Liquor.

With the Kraft process, 85 to 93% of the lignin and 56 to 71% of the hemicelluloses present in the wood are removed and the degradation products of these substances constitute the major non-aqueous content of Black Liquor. Typical black compositions are between 12 and 18% solid content. Crude turpentine is recovered from the digester drain and evaporator condensates, and most of the oily substances are removed during the evaporation process of the Black Liquor by skimming.

Due to its strong binding, the presence of LCC affects the overall extraction of lignin and carbohydrates. For example, a low yield of the Kraft pulping process is related to the difficulty of breaking lignin-carbohydrate bonds in hardwood species, which can be attributed to the alkaline stability of the LCC bond type in hardwoods. In addition, the formation of new lignin-hemicellulose bonds in the Kraft extraction process has been hypothesized.

### ORGANOSOLV PROCESS

In industrial papermaking processes, Organosolv is a pulping technique that uses an organic solvent to solubilize lignin and hemicellulose. It has been considered in the context of both pulp and paper manufacturing and biorefining for the subsequent conversion of cellulose into fuel ethanol.

Organosolv has several advantages over Kraft or sulphite pulp. In particular, the ability to obtain relatively high-quality lignin adds value to a process flow that would otherwise be considered waste. Organosolv solvents are easily recovered via distillation, which results in less water pollution and the elimination of the odor associated with sodium sulfide used in the Kraft process.

### SODA PROCESS

To give an order of magnitude of the total market, FAO reports that global wood pulp production in 2023 was about 193 million tons. Within this total, firm paste is a niche; moreover, non-wood pulp worldwide is often estimated to be in the order of a few million tons per year, not tens of millions. So the most honest estimate is: cellulose produced via soda is now a small share of the world pulp total, probably concentrated in non-wood and specialty applications, not in large commodity paper.

In the soda process, the main reagent is NaOH. The alkaline environment first of all ionizes phenolic and carboxyl groups of lignin, increasing its solubility; then it mainly promotes the cleavage of aryl-ether bonds, in particular β-O-4, which are the most abundant and most important bonds in the lignin network. This leads to smaller, more phenolic, and more soluble lignin fragments in black liquor. The same alkaline environments can also break part of the lignin-carbohydrate bonds, especially esters and part of the benzyl-ethers, freeing or shortening the LCC complexes.

In addition to the lignin-hemicellulose bonds, those inside the lignin are also broken, but not as easily. In the soda process, the ether bonds inside the lignin are broken well, especially the β-O-4; many LCC bonds are also broken, especially the more labile ones such as esters and a part of the lignin-carbohydrate ether bonds. In contrast, the condensed C-C bonds of lignin are much stronger and remain largely intact. For this reason, the final product is not only made up of monomers: usually a mixture of lignin oligomers is obtained, plus a share of residual LCCs.

Cellulose, on the other hand, resists better because it is a linear, crystalline and much neater structure. In soda pulping, the goal is precisely to remove as much lignin as possible while preserving the cellulose fiber as much as possible; for this reason, soda works well especially on biomass that is easier to denigify, such as straw and other non-woods. In short: you don't "dissolve" the cellulose, but you make the lignin more and more soluble and fragmented until it separates from the fiber.

### CHEMICAL CHARACTERISTICS OF BLACK LIQUOR

The composition of Black Liquor depends characteristically on the wood species and the delignification process and the operating conditions used. Typically, softwood Black Liquor contains more lignin and fewer aliphatic carboxylic acids (especially acetic acid) and other organics (especially xylan residues) than hardwood Black Liquor.

The dry residue obtained from the precipitation of solid substances after acidification of Black Liquor at a pH of around 2, consists of 25 to 35% lignin and its derivatives, 30 to 35% polysaccharides, 5 to 10% other organic substances (i.e. organic residues derived from hemicelluloses and extractable, meaning substances of protein or oil origin or their derivatives present in wood and extracted from the basic solutions used for and 30 to 40% of inorganic substances (i.e. chemical cooking residues and sodium and sulphur bound to organic substances).

Lignin-carbohydrate complexes (LCCs) can be extracted and dissolved together with lignin and hemicelluloses during biomass delignification processes. During treatment with the alkaline solution, LCCs are partly degraded.

The molecular weight (MW) of LCCs and lignin depends on the type of biomass and the extraction process used. Based on the evaluations made by some studies and considering a typical extraction where LCCs represent about 25% of the total mass extracted and lignin frees 75%, here are in the following table 2 estimated average values for the total complex.

**TABLE 2**

| Composition Black Liquor from softwood. Kraft Process | | | |
|---|---|---|---|
| Biomass | Free Lignin Mw in Da | LCC Complex Mw in Da | Estimated total Mw in Da |
| Hardwood | 20.000 | 40.000 | 25-30.000 |
| Softwood | 8.000 | 140.000 | 40-50.000 |
| Woody plants | 3.000 | 12.000 | 5.000-7.000 |

It is necessary to warn that these values are extremely sensitive to the extraction method. If a Kraft process or strong acid hydrolysis is used, the LCC bonds are almost totally cleavaged, leading the total Mw to coincide almost exclusively with that of the fragmented lignin, thus obtaining much lower values, around 3,000-5,000 Da).

The molecular weights reported in scientific studies are almost always referred to the precipitated lignin and subsequently resolubilized for GPC/SEC chromatographic analysis, and not directly to the Black Liquor as it is. The technical and procedural reasons for operating in this way are due to interferences in the Black Liquor, such as inorganic salts (caustic soda, sulphides, carbonates) in high concentrations, degraded hemicellulose, iso-saccharinic acids, extractives and soaps. These components create an unacceptable noise floor for GPC (UV or Refractive Index) detectors and would quickly damage chromatographic columns.

To obtain reliable data, the standard procedure follows these steps:
- precipitation: the Black Liquor is acidified (usually at pH 2) to precipitate the lignin and LCC complexes;
- washing and drying: salts and soluble carbohydrates are removed;
- resolubilization: dry lignin is dissolved in a solvent suitable for GPC, the most common are THF (Tetrahydrofuran) which often requires preventive acetylation of lignin to make it soluble, DMF (Dimethylformamide) + LiBr widely used because it dissolves lignin well without derivatization.

It is crucial to note that the molecular weight measured is not that of the "native" lignin in the wood, but that of the lignin fragmented by the cooking process (e.g., Kraft or Soda). The Kraft process breaks the β-O-4 bonds, drastically lowering the Mw compared to that of the plant. So, for what has been written above, most of the literature articles describe the technical lignin obtained after recovery from lye. If you measured Black Liquor directly (assuming you have resistant columns), you would see a molecular profile distorted by the presence of monomers, dimers and heavy salts.

In the article "Lignin-Derived Chemicals from Pulping" published by NREL, the average molecular weight of lignin and LCC actually contained in the Black Liquor of wheat straw and switchgrass (*Panicum virgatum*) is measured by GPC with LC-UV280nm chromatogram. The results of average molecular weight (Mw), numerical mean molecular weight (Mn) and polydispersivity (Pd) are shown in table 3 below.

**TABLE 3**

| Molecular Weights from Wheat Straw and Swithgrass | | | |
|---|---|---|---|
| | Mw | Mn | PD |
| Wheat Straw | 1400 | 510 | 2,8 |
| Switchgrass | 600 | 340 | 1,8 |

The numerical difference between Mw and Mn is related to polydispersivity, the higher Pd, the greater the difference between Mw and Mn.

The results of the study in the article "Lignin-Derived Chemicals from Pulping" indicate that the composition of lignin and LCC present in Black Liquor is different from that of precipitated lignin. The acidification treatment involves the precipitation of soluble or emulsifiable macromolecules in a basic environment, while a significant part of polyphenols with lower molecular weights and polyphenols bound to highly hydrophilic polysaccharide fragments (LCCs) remain solubilized in the aqueous phase.

Carbohydrates can be present in Black Liquor not only as lignin-related complexes but also as monomer units or polysaccharides as such. The origin of most of these compounds is the hemicellulose component, which is more water-soluble, generally of lower mass, and lacks the largely crystalline character of cellulose. Black Liquors from the Kraft pulp of *Eucalyptus globulus* contain between 2.9 and 7.3% polysaccharides in reference to the precipitated dry solid. To get an idea of the molecular weight of these polysaccharides, an average molecular weight of the xylan component (which is the main component) of about 18,000 g/mole has been calculated in eucalyptus.

A significant aliquot of Black Liquor carbohydrates is bound to lignin in lignin carbohydrate complexes (LCCs). Indicatively, the LCC fraction in herbaceous biomass represents 10-30% wt of the lignin fraction, in hardwood it is 5-20% wt and in softwood it is 5-15% wt.

The availability of extractive compounds in a pulp production plant is highly dependent on the wood species used for pulp production, the method and time of storage of logs and chips, and the growing conditions of the trees. For example, the typical extract content in native softwoods is less than 5% of the dry solids of the wood. Of these, about 40% consists of resinic acids (e.g. abietic and pymaric acid), about 10% of free fatty acids (e.g. oleic, linoleic and pinolenic acid), about 40% of fatty acid esters (e.g. fats and waxes) and about 10% of other fatty acid esters (e.g. mostly neutral substances, "unsaponifiable"). In contrast, hardwoods from temperate zones normally contain less than 4% of the dry solids of wood, and this fraction consists of about 20% free fatty acids, about 55% fatty acid esters (fats), and about 25% others. During kraft paste, the volatile components of turpentine are chemically stable, but fatty acid esters are hydrolyzed almost completely. Because of this saponification with the simultaneous neutralization of aliphatic carboxylic acids, extractives also consume cooking chemicals.

Lignin is rich in inorganic salts, which come mainly from the so-called White Liquor which is the alkaline solution used to extract lignin from wood and acids used for neutralization after treatment. In the Kraft process, sodium sulfide Na₂S is also used together with solutions of sodium hydroxide NaOH.

Table 4 below shows the analytical characteristics of diluted Black Liquor obtained from softwood (80% wt spruce, 20% wt pine) and produced by a Swedish company that produces pulp with the Kraft process. The source of the data is the article "Precipitation of Lignin from Softwood Black Liquor: Investigation of the Equilibrium and Molecular Properties of Lignin (Weizhen Zhu and Hans Theliander)".

**TABLE 4**

| Composition Black Liquor from softwood. Kraft Process | |
|---|---|
| Feature | Content (g/I) |
| Lignin UV | 118,5 |
| Lignin Klason | 98,8 |
| Carbohydrates | 5,5 |
| NaOH (sodium hydroxide) | 11,2 |
| Na2S (sodium sulfide) | 14,7 |
| Na (sodium) | 61,5 |
| K (potassium) | 7,7 |
| Total Solids Content 32% wt | |

The concentration of lignin was determined by absorption of UV light using a Specord 205, Analytik Jena, with a wavelength of 280 nm, while the Klason lignin in the sample is the solid material left over after hydrolysis treatment with 72% sulfuric acid. Klason lignin is lower because it measures only the one that has precipitated, the remaining part is solubilized or degraded after acid treatment.

Table 5 below shows the typical composition and chemical and physical characteristics of Black Liquor obtained from wheat straw using the Phoenix Process of Sustainable Fiber Technologies (SFT). This process does not use sulphides.

**TABLE 5**

| Composition Black Liquor from wheat straw Soda Process | | |
|---|---|---|
| Feature | Units | Value |
| Lignin UV | g/l | 100 - 200 |
| Inorganic salts | g/l | 150 - 200 |
| Carbohydrates | g/l | 2 - 8 |
| Protein compounds | g/l | 3 |
| Fats | g/l | 2 |
| Sodium Na+ | g/l | 25 |
| Potassium K+ | g/l | 2,6 |
| Calcium Ca ²⁺ | g/l | 1,3 |
| Total solids | % | 35-50 |
| pH | - | 9-11 |
| Viscosity | cp | < 500 |

The Black Liquor which must be used in cosmetics can possibly be subjected to purification treatments, separation of unwanted components such as sulphides present in the Black Liquor of the Kraft process, or lipid impurities.

Sulphide purification of lignin can be carried out by stripping H₂S hydrogen sulfide in a vacuum at a temperature not exceeding 30-40 °C and after slightly lowering the basic pH to values not lower than 8. At this pH the presence of H₂S deriving from the protonation of sulphides is already significant and the precipitation of lignin polyphenols is avoided.

Lipid impurities that remain dispersed in the Black Liquor and tend to separate over time can be separated by centrifugation.

The following examples show the methods of preparation of some cosmetic compositions with additives with Black Liquor and on some of them the benefits obtained when increasing quantities of Black Liquor are added to a given composition have been evaluated.

The type of Black Liquor to be used as the reference for the examples was the one obtained from wheat straw using the Phoenix Process^{™}, developed by Sustainable Fiber Technologies (SFT), which actually uses a process based on soda (sodium hydroxide) for the extraction of lignin.

Unlike more common industrial methods such as the Kraft process, the Phoenix Process is designed to be a more sustainable and environmentally friendly alternative, focused on the valorization of non-wood biomass (such as wheat straw, agricultural residues and bamboo).

A characterization of Black Liquor from Wheat Straw produced by SFT was presented in the article "Lignin-Derived Chemicals from Pulping Liquors" published by NREL. Gel Permeation Chromatography (GPC) was used to determine the molecular weights, which showed that:

| | |
|---|---|
| Average molecular weight Mw | = 1400 Da |
| Average molecular weight Mn | = 510 Da |
| Polydispersivity | = 2.8 Da |

Thus, it can be observed that the average molecular weights of the Black Liquor from Wheat Straw in this example are much lower than those of the precipitated lignin, as the soluble molecular low weight fractions are missing.

### EXAMPLE 1

### Evaluation of emulsifying properties Black Liquor

Two cleansers have been prepared where glycerin saturated chain fatty acid esters Glyceryl Palmitate and Glyceryl Laurate are used as components, and a Black Liquor obtained from straw supplied by the SFT company, the composition of which is shown in table 6 below.

**TABLE 6**

| Composition Black Liquor Example 1 | | |
|---|---|---|
| Feature | Units | Value |
| Lignin | g/l | 130 |
| Inorganic salts | g/l | 160 |
| Carbohydrates | g/l | 5 |
| Protein compounds | g/l | 2 |
| Fats | g/l | 1 |
| Sodium Na+ | g/l | 20 |

The composition of the cleansers, one called GP and the other GL is shown in table 7 below.

**TABLE 7**

| Composition Cleansing- Make up remover | | |
|---|---|---|
| Component | GP % weight | GL % weight |
| Glyceryl Palmitate | 5 | |
| Glyceryl Laurate | | 5 |
| Black Liquor by Table 6 | 10 | 10 |
| Glycerin | 3 | 3 |
| Xanthan gum | 1 | 1 |
| Water | 81 | 81 |
| Total | 100 | 100 |

The methods of preparation of the emulsion were as follows. The aqueous phase was heated up to 70-80°C. Then the Black Liquor was added to the aqueous solution under continuous stirring. Xanthan Gum was then slowly dispersed in the solution for a quantity equal to 1% wt and left to hydrate for 1 hour under mixing. Subsequently, glycerin and ester were added. Everything was mixed for others for about 10 minutes. At the end the emulsion was transferred to the storage container.

From the microphotographs that were taken, the emulsion was stable. One month after preparation, the emulsion remained stable and no separate phases were noticed.

### EXAMPLE 2

### Cleansing/Make up remover containing lignin from Black Liquor

A cleanser with make-up removing properties was prepared using among the components a Black Liquor obtained from straw supplied by the company SFT whose composition is shown in Table 5.

The composition of the Cleansing-Make Up Remover is shown in Table 8 below.

**TABLE 8**

| Composition Cleansing- Make up remover | |
|---|---|
| Ethyl hexyl palmitate | 15 g |
| Black Liquor by Table 6 | 10 g |
| Glycerin | 5 g |
| Carboxy-vinyl polymer | 0.5 g |
| Sodium hydroxide 50% | 0.2 g |
| Preservatives | - |
| Water | 69,3 g |
| Total | 100 g |

The other ingredients are Ethyl hexyl palmitate supplied by Sigma Aldrich, Glycerin supplied by Chimitex, carpoxyvinil polymer marketed by Lubrizol under the name Carbopol Ultrez 10 NF. The Cleanser has been prepared in the following way. They are added to a turbo-mixer under agitation and in sequence: water, Black Liquor, ethyl hexyl palmitate, heat up to about 50°C and then gradually and cautiously add Carbopol. It is then neutralized with a 50% aqueous solution of sodium hydroxide NaOH and left under agitation for about 10 minutes. At the end, stirring stops and the emulsion is transferred to the storage container.

### EXAMPLE 3

### Properties such as Make-up remover from Example 2

The cleanser with the ability to remove make-up components must not only have the ability to remove oily substances deposited on the surface of the skin, but must also have dispersing abilities, so as to be able to remove nanoparticles of titanium dioxide or iron oxide or other metal oxides that are part of make-up.

To evaluate the properties of lignin and lignin-carbohydrate complexes (LCC) contained in Black Liquor as a cleanser and make-up remover, a comparison was made between the composition of Example 2 with the commercial make-up remover micellar milk produced by Venus, containing as ingredients: Ethylhexyl Palmitate, Glycerin, Polysorbate 60, Sorbitan Stearate Prunus Dulcis Oil, Ceteareth-20, Decyl Glucoside, Hamamelis Virginiana, Panthenol, Carbomer, Parfum, Phenoxyethanol, Ethylhexyl Glycerin, Sodium.

Venus' commercial product contains, as surfactants and non-ionic dispersants Polysorbate 40, Sorbitan stearate, ceteareth-20, Decyl Glucoside.

A fixed amount of 2 grams of a commercial foundation was distributed on the inside of a volunteer's arm and gently distributed on a surface of about 3 cm x 6 cm with the tip of the finger after wearing a disposable glove in order to form a veil. Then, in the center of the rectangle, add 5 ml of the cleanser and distribute it on the surface of the treated skin and in the same way as seen above, distribute the cleanser on the treated surface for 10 seconds, (in order to simulate the normal use of the cleanser by the user). Then, you remove everything using a certain number of cotton pads by sliding them once along the longest direction of the rectangle. In this way, a part of the foundation deposited on the skin is removed from the cotton pad. With each pass, the amount of foundation is reduced and since it has a color between beige and brown, the extent of its removal is easily identifiable visually. Continue in this way until only traces of foundation visible on the surface of the pad appear from the examination.

The test carried out shows that 8 passes with the cotton pad were necessary for both cleansers to obtain the desired result.

From the comparison between the two cleansers it can be seen that both contain the emollient ingredient such as Ethylhexyl Palmitate and the gelling ingredient such as Carbomer, but the commercial product of Venus contains, as surfactants and non-ionic dispersants Polysorbate 40, Sorbitan stearate, ceteareth-20, Decyl Glucoside, while with the composition of the invention the ingredient with dispersing properties is necessarily the Black Liquor component, which contains lignin and the lignin-carbohydrate complexes.

### EXAMPLE 4

### Depilatory cream with Black Liquor instead of surfactants

To perform a proper shave, two conflicting needs must be met: on the one hand, the skin must be lubricated to avoid grazes during the sliding of the blade and on the other hand, the hair of the beard must be wet in order to make it easier to cut.

The problem is that the lubricant exerts its action not only on the surface of the skin, but also on that of the hair, making it difficult to cut. A reduction in lubrication makes the coat wetter but increases the possibility of cuts on the skin.

Polymeric lubricants, consisting of long chains that stretch like overlapping bundles not only on the skin, but also on the hair, have the disadvantage of forming a continuous protective layer that is undoubtedly efficient from the point of view of lubrication, but which makes it difficult to cut the hair, because the lubricant also adheres to that surface.

In addition, surfactants must also be included in the formulation of the shaving cream or soap, which have the dual function of both promoting the formation of stable emulsions between the lipophilic and hydrophilic components of shaving creams, and of facilitating, with the final rinse at the end of shaving, the effective removal of the layer of lubricant and other substances such as dirt and in general residues of degradation of insoluble or poorly soluble bio-molecules in water.

An example of a depilatory cream where Black Liquor has been added to replace any type of anionic, non-ionic and cationic surfactants normally used in shaving creams.

The composition of the depilatory cream is shown in Table 9 below.

**TABLE 9**

| Surfactant-free composition | |
|---|---|
| Glyceryl Palmitate | 5 g |
| Black Liquor by Table 6 | 10 g |
| Glycerol | 5 g |
| Xhantan Gum | 1.2 g |
| Preservatives | - |
| Water | 78,8 g |
| Total | 100 g |

The depilatory cream has been prepared in the manner described in Example 1. The application on the surface to be shaved is done by spreading the cream evenly, using the spatula provided or your fingers.

The emulsion formed was stable three months after preparation without any signs of separation of any component.

The shaving tests that have been carried out with the composition of Table 8 have given a satisfactory result as it appears that: a) the razor is able to slide by cutting the hair and this means that a layer of oily lubricant has not formed around the hair that makes the razor slide over it preventing it from being cut; b) the shave was carried out without any cuts or redness on the skin and this indicates that the lubrication of the skin was sufficient to protect the skin from penetration by the razor blade.

### EXAMPLE 5

### Depilatory cream with only Black Liquor and lipid esters

An example of the use as a depilatory cream of a composition where lignin and the lignin-carbohydrate complexes contained in Black Liquor perform the typical functions of surfactants and ingredients with emulsifying, humectant, stabilizing, thickening and dispersing properties is reported.

The cream contains only lipids and Black Liquor and the composition is shown in table 10 below.

**TABLE 10**

| Composition with only lipids and Black Liquor | |
|---|---|
| Glyceryl Laurate | 15 g |
| Black Liquor by Table 3 | 40 g |
| Surfactants | - |
| Stabilizers, moisturizers | - |
| Preservatives | - |
| Water | 45 g |
| Total | 100 g |

Also in this case satisfactory results have been obtained. The composition was more viscous than the composition of Example 3 and this allowed to favor the formation of a thicker layer of cream and therefore more lubricated and more protective. This has made it possible to make shaving smoother without penalizing the ability to cut because lignin and LCC probably also have the ability to hydrate the hair and this is a factor that helps cutting.

### EXAMPLE 6

### Antibacterial properties of compositions containing Black Liquor and glyceride esters towards Gram +

In this example, the antimicrobial properties against Gram + *Straphilococcus Epidermis* and *Straphilococcus Aureus bacteria of* some formulations of cleansers or make-up removers for the skin containing Black Liquor obtained from wheat straw supplied by the company SFT were evaluated. The composition of the Black Liquor used in this test is shown in Table 6. In addition to Black Liquor in concentrations of 10 and 15% wt, the formulation of the cleanser/make-up remover contains: Glyceryl Laurate, Glycerin, Xhantan Gum and sodium benzoate.

The formulations of the compositions subjected to the antimicrobial evaluation, whose pH was between 5.5-6 by acidification with lactic acid, are shown in Table 11 below.

**TABLE 11**

| Compositions tested for antibacterial activity | | | | | |
|---|---|---|---|---|---|
| | Black Liquor | Glyceryl Laurate | Glyceryl | Xhantan Gum | Water |
| | % wt | % wt | % wt | % wt | % wt |
| BL10-GL08 | 10 | 8 | 3 | 1 | q.s. 100 |
| BL10-GL6.5 | 10 | 6,5 | 3 | 1 | q.s. 100 |
| BL10-GL05 | 10 | 5 | 3 | 1 | q.s. 100 |
| BL15-GL05 | 15 | 5 | 3 | 1 | q.s. 100 |

The study was performed with the "Broth Microdilution Assay". The standard medium used is Mueller-Hinton broth, which is recommended for fast-growing aerobic bacteria.
The typical composition of the broth is as follows:
- hydrolysed casein acid approx. 17.5 g/L
- starch about 1.5 g/L,
- meat extract/infusion 2-3 g/L
Typical test operating conditions are:
- 1:2 serial dilutions of the sample in the broth,
- final inoculation about 5 × 10^5 CFU/mL,
- incubation at 35 ± 2 °C for about 16-20 hours.
The measurement is based on the determination of the Minimum Inhibition Concentration MIC, which is the minimum amount of substance inoculated into the broth that allows the absence of visible growth or turbidity in the broth. MIC was expressed in mgr/ml.

The results of Minimum Inhibition Concentration (MIC) against gram + S. *epidermis* and *S. aureus* bacteria are shown in table 12 below.

**TABLE 12**

| Anti-bacterial activity test results | | |
|---|---|---|
| | S. Epidermis mg/gr | S. Aureus mg/gr |
| BL10-GL08 | 0,39 | 6,2 |
| BL10-GL6,5 | 0,78 | 6,2 |
| BL10-GL05 | 1,56 | 12,5 |
| BL15-GL05 | 0,39 | 3,1 |

In *S. Epidermis ,* the MIC of the BL10-GL05 formulation is 1.56 mgr/gr, while that of GL05/BL15 is 0.39 mgr/ml. This means that in the face of a 50% increase in BL concentration, the MIC dropped 4 times.

The same trend was also observed with *S. Aureus,* but starting from higher MIC values than with *S. Epidermis.* The MIC of the BL10-GL05 formulation is 12.5 mgr/gr, while that of BL15-GL05 is 3.1 mgr/gr. This means that a 50% increase in BL concentration resulted in a huge 4-fold MIC reduction, as in the case of *S*. *Epidermis.*

The results obtained reveal an interesting anti-microbial activity towards the gram-positive bacteria of the formulations under investigation. And even if it is proven that Glyceryl Mono-laurate has an intrinsic anti-microbial activity against gram+ bacteria, the fact that an increase in the concentration of Black Liquor by 50% wt leads to a marked reduction of 400% in abatement efficiency, demonstrates the strong adjuvant and synergistic action of Black Liquor.

To get a quantitative idea of the activity of these formulations towards gram+ bacteria, the data of MIC = 3.1 mgr/ml for *S. Aureus* referred to the BL15-GL05 composition is taken as an example and compared with the results of similar tests performed with other polyphenols on the same *S. Aureus.*

In the BL15-GL05 composition there was 15 % wt of Black Liquor and 5 % wt of Glyceryl Laurate. However, the fraction of Glyceryl mono-laurate (GML) was made up of 55% wt, while the remaining 45% was made up of Glycerol di-laurate (GDL), so the real concentration of anti-microbial GML was around 2.5%.
If MIC = 3.1 mgr/mL is considered, there are approximately the following concentrations in the broth:
- BL = 15% wt of 3.1 mg/mL = 0.465 mg/mL
- if the lignin in BL is 12% → apparent lignin = 0.0558 mg/mL = 55.8 µg/mL
- GL = 5% of 3.1 mg/mL = 0.155 mg/mL
- if 50% is GML → apparent GML = 0.0775 mg/mL = 77.5 µg/mL
So it can be admitted that the concentration of polyphenols that make up lignin is approximately 55.8 µg/mL.

Making a comparison with some polyphenols whose anti-oxidant activity is known, the article "Antimicrobial Activities of Natural Bioactive Polyphenols" (Manas Kumar Mandal and Abraham J. Domb) evaluates the antimicrobial activities of some of the most well-known polyphenols. The data are provided in IC50, which refer to the concentration of the substance inoculated into the broth which allows a 50% reduction in bacterial growth. MIC is more restrictive because it refers to the concentration in which growth is totally inhibited.

The article reports these values of IC50 towards *S. Aureus*:
- 4-Hydroxybenzoic acid: IC50 = 926 µg/mL
- Catechin: IC50 = 5.65 µg/mL
- Quercetin: IC50 = 19.6 µg/mL (65 µM, PM = 302.24).
The MIC value of 55.8 µg/mL found for the BL15-GL05 formulation indicates that this composition is much more potent than 4-Hydroxybenzoic acid and is comparable to Quercetin although less potent. On the other hand, Catechin is 10 times more effective than BL15-GL10. However, the fact that this activity was obtained with a crude lignin mixture, not with a pure polyphenol, is nevertheless a remarkable result. In fact, we must consider that pure polyphenols such as Quercetin are obtained through complex isolation systems, while the lignin polyphenols of Black Liquor are found in the basic solution immediately after the attack with sodium hydroxide without the need to proceed with complex isolation processes to obtain the anti-microbial agent.

It should also be borne in mind that data were provided for formulations containing 10 and 15 % wt BL, with variable GL contents and with a fixed fraction of Xhantan Gum of 1 % wt and glycerine of 3 % wt. By optimizing the fractions of these components, it is possible to further improve the anti-microbial activity of these compositions. Thus it can be said that a mixture of relatively dilute lignin oligophenols, made more active by the interaction of lipid esters such as Glyceryl Laurate, achieves an overall antimicrobial activity of the same order of magnitude as some natural polyphenols known for their efficiency. This is already a very interesting result because it allows us to find a useful and wide use in the field of hygiene and skin care for polyphenols that are obtained from the extraction of lignin from wood.

The same Broth Microdilution Assay test, performed on samples of Black Liquor as it is at 10% wt and 15% wt, gave as a result MIC > 200 mgr/ml and this means no anti-microbial activity.

### EXAMPLE 7

### Antioxidant properties of formulations with Black Liquor and glyceride esters

The anti-oxidant properties of some compositions containing Black Liquor in Table 3 were evaluated together with the glyceride esters Glyceryl Laurate (GL), Glyceryl Palmitate (GP) and Glyceryl soyate (GS), obtained from soy fatty acid. The pH was adjusted to slightly acidic values with benzoic acid. The fractions of the components are shown in Table 13 below.

**TABLE 13**

| Compositions tested for antioxidant activity | | | | | | |
|---|---|---|---|---|---|---|
| | Ester type | Black Liquor | Esther | Glyceryl | Xhantan Gum | pH |
| | | % wt | % wt | % wt | % wt | |
| BL10-GL05 | Laurate | 10 | 5 | 3 | 1 | 6,5 |
| BL10-GP05 | Palmitate | 10 | 5 | 3 | 1 | 6,8 |
| BL10-GS05 | Soyate | 10 | 5 | 3 | 1 | 6,5 |

Together with the compositions indicated in Table 12, the anti-oxidant power of the following components as such was also evaluated. To evaluate the anti-oxidant power, the TBA assay (or thiobarbituric acid test) was adopted. This is a classic and widely used method, particularly for its ability to inhibit lipid peroxidation. When a % LPI value is reported in the context of a TBA assay, this typically indicates the percentage of "Lipid Peroxidation Inhibition", i.e. the percentage of inhibition of lipid peroxidation due to the presence of the anti-oxidant tested.

The results obtained on the substances as they are shown in Table 14 below:

**TABLE 14**

| TBA assay results on substances as they are | |
|---|---|
| Substance | % LPI |
| Black Liquor | + 17 |
| Glyceryl Laurate | + 20 |
| Glyceryl Soyate | + 16 |
| Glyceryl Palmitate | -20 |

To interpret the data in Table 14, we take into account the fact that %LPI values between 10 and 30 % indicate low anti-oxidant activity, while between 30 and 60 % indicate moderate but significant anti-oxidant activity. Instead, negative values indicate pro-oxidant activity. Table 15 below shows the typical values of some polyphenols and a powerful antioxidant such as alpha-tocopherol in a concentration of 1% wt.

**TABLE 15**

| TBA assay results of typical anti-oxidants | |
|---|---|
| Substance | % LPI (range) |
| Pure standard Quercetin | 35-60 |
| Pure standard catechin | 30-50 |
| Alpha-Tocopherol 1% wt | 20-35 |

The result of %LPI of +17 of Black Liquor is interesting because the concentration of polyphenols that make up lignin is typically 12 % wt. Ideally, reporting the data obtained at 12% wt of 100% lignin, would have resulted in %LPI = 142, which is a very high value and about 3 times higher than Quercetin and Catechin.

Among other pure substances, Glyceryl Laurate and Glyceryl Soyate showed low activity, while Glyceryl Palmitate showed negative activity, meaning that it behaves like a pro-oxidant.

Table 16 below shows the results obtained with the compositions subject to this study.

**TABLE 16**

| TBA assay results on compositions | |
|---|---|
| Composition | % LPI |
| BL10-GL05 | +33 |
| BL10-GP05 | -10 |
| BL10-GS05 | +28 |

The data in Table 16 referring to compositions with 10% Black Liquor and 5% Glyceryl Laurate and Soyate indicate that the presence of these esters in the formulation of the cosmetic composition synergistically emphasizes the anti-oxidant properties of Black Liquor. In fact, for example LPI = 33% of BL10-GL05 is within the typical values of anti-oxidants such as Catechin and Quercitin. On the other hand, the presence of Glyceryl Palmitate in the formulations results in the formation of an emulsion that lacks anti-microbial properties.

In the BL10-GL05 composition, lignin is at a concentration of 1.2% wt and Glyceryl Laurate is at 5% wt and the result of +33% LPI is much higher than the result of +17% of Black Liquor as it is (with 12% wt of lignin). The same goes for Glyceryl Laurate which, as such, has an LPI = 20 %. Evidently there is a synergistic action between GL and lignin polyphenols that emphasizes the anti-oxidant properties of both.

### NEW PARADIGM OF COSMETICS

Examples 1 to 5 have shown that the components of Black Liquor, consisting of lignin polyphenols, but also lignin bound to polysaccharide fragments (LCC), are able to confer emulsifying, detergent and dispersant properties that make Black Liquor itself a suitable alternative cosmetic ingredient to other ingredients of synthetic origin.

The experimental results of Example 6 indicate that emulsions or other types of mixtures containing Black Liquor and esters of Glyceryn have good anti-microbial activity towards the gram+ bacteria *S*. *Epidermis* and *S. Aureus.* On the other hand, the results of the tests conducted on Black liquor diluted in water up to 10 and 15 % did not show anti-microbial action. Fatty acid esters probably aggregate to the polyaromatic polar groups of lignin, favoring contact with the lipid membrane of bacteria and subsequent absorption into it.

No antimicrobial properties of emulsions containing Black Liquor and other esters have been tested, but it is presumable, on the basis of what has been observed in example 7, that other fatty acid esters such as oleic or linoleic may play the same role as "carriers" of polyphenols within the bacterial membrane.

No tests have been done on anti-microbial properties on Gram- bacteria, but information in the literature reports numerous studies confirming the antimicrobial properties of lignin against Gram- bacteria such as *Escherichia coli,* although its efficacy is generally lower than that shown against Gram- bacteria.

Example 7 showed that cosmetic compositions containing, in addition to Black Liquor, certain fatty acid esters, have interesting anti-oxidant properties. These properties were not shown when a palmitic acid ester such as Glyceryl Palmitate was used. Therefore, these properties are activated when substances such as certain lipid esters are present in the formulation of the cosmetic capable of promoting the penetration of antioxidant polyphenols into the stratum corneum.

Ultimately, Black Liquor is of natural origin and not harmful. When added to hygiene and skincare formulations, it helps maintain the integrity of the skin as it is free of surfactants and protects the skin from the deleterious action of bacteria and free radicals on the skin.

In addition to being natural and easily available, the use of Black Liquor in cosmetic compositions makes it possible to avoid many compounds of synthetic origin with toxic characteristics, such as anionic surfactants.

## Claims

1. Use of Black Liquor as an ingredient in cosmetic compositions.

2. Use according to claim 1, in which Black Liquor is an aqueous mixture that is obtained from any process of extraction of cellulose from biomass containing lignin.

3. Use according to claim 2, in which the extraction process is chosen from Kraft, Soda Pulping, Sulphite, Organosolv.

4. Use according to claim 2 or 3, where the process includes the treatment of hardwoods, soft wood preferably obtained from conifers, wheat straw, woody plants and grasses.

5. Use according to any of claims 1 to 4, where Black Liquor has a lignin content present as polyphenols of at least 0.1% wt on the total weight of the liquid.

6. Use according to claim 5, in which lignin is present both in the free form of polyphenols and in the form of LCC lignin-carbohydrate complexes.

7. Use according to any of claims 1 to 6, where Black Liquor has cleansing, make-up-removering, anti-aging, preservative, emulsifier, dispersant, antioxidant, antimicrobial properties.

8. Cosmetic composition including Black Liquor as an ingredient where its concentration is at least 1 % by weight of the total composition as it is.

9. Cosmetic composition according to claim 8, for use as a cleanser, shampoo, makeup remover, hair removal cream, shaving cream, beauty cream, anti-aging cream.
